# EUROPEAN PATENT SPECIFICATION

(11) **EP 2 074 147 B1**
(45) Date of publication and mention of the grant of the patent: **21.03.2012**
(21) Application number: 07818397.7
(22) Date of filing: 25.09.2007
(51) Int. Cl.: C07K 16/28, C12N 15/13, A61K 39/395, A61P 37/00, A61P 31/18

(54) **ANTIBODIES AGAINST CCR5 AND USES THEREOF**
ANTIKÖRPER GEGEN CCR5 UND IHRE VERWENDUNG
ANTICORPS DIRIGES CONTRE LE CCR5 ET LEURS UTILISATIONS

(30) Priority: 29.09.2006 EP 06020646
(43) Date of publication of application: 01.07.2009
(73) Proprietor: F.Hoffmann-La Roche AG, 4070 Basel (CH)
(72) Inventor: AUER, Johannes, 82445 Schwaigen (DE); BAEHNER, Monika, 81379 München (DE); BRANDT, Michael, 82393 Iffeldorf (DE)
(74) Representative: Burger, Alexander
(86) International application number: PCT/EP2007/008312
(87) International publication number: WO 2008/037419

(56) References cited:
- WO-A-88/07089
- WO-A-2005/105841
- WO-A-2006/103100
- US-B1- 6 528 625
- OLSON W C ET AL: "Differential inhibition of human immunodeficiency virus type 1 fusion, gp120 binding, and CC-chemokine activity by monoclonal antibodies to CCR5" JOURNAL OF VIROLOGY, THE AMERICAN SOCIETY FOR MICROBIOLOGY, US, vol. 73, no. 5, May 1999 (1999-05), pages 4145-4155, XP002290005 ISSN: 0022-538X cited in the application
- STEINBERGER P ET AL: "GENERATION AND CHARACTERIZATION OF A RECOMBINANT HUMAN CCR5-SPECIFIC ANTIBODY A PHAGE DISPLAY APPROACH FOR RABBIT ANTIBODY HUMANIZATION" JOURNAL OF BIOLOGICAL CHEMISTRY, AMERICAN SOCIETY OF BIOLOCHEMICAL BIOLOGISTS, BIRMINGHAM,, US, vol. 275, no. 46, 17 November 2000 (2000-11-17), pages 36073-36078, XP000999141 ISSN: 0021-9258 & DATABASE EMBL [Online] 11 November 2000 (2000-11-11), "Oryctolagus cuniculus anti-human CCR5 immunoglobulin light chain variable region mRNA, partial cds." retrieved from EBI accession no. EMBL:AF281659 Database accession no. AF281659
- "Human Genome Sciences will start clinical trials with anti-CCR5 monoclonal antibody." IAVI REPORT : NEWSLETTER ON INTERNATIONAL AIDS VACCINE RESEARCH 2004 DEC-2005 MAR, vol. 9, no. 1, December 2004 (2004-12), page 15, XP008075310 ISSN: 1816-6253
- FOR THE PEDIATRIC AIDS CLINICAL TRIALS GROUP PROTOCOL 351 STUDY GROUP SHEARER ET AL: "Susceptibility of pediatric HIV-1 isolates to recombinant CD4-IgG2 (PRO 542) and humanized mAb to the chemokine receptor CCR5 (PRO 140)" JOURNAL OF ALLERGY AND CLINICAL IMMUNOLOGY, MOSBY - YEARLY BOOK, INC, US, vol. 118, no. 2, August 2006 (2006-08), pages 518-521, XP005587373 ISSN: 0091-6749
- ZHANG JUN ET AL: "The second extracellular loop of CCR5 contains the dominant epitopes for highly potent anti-human immunodeficiency virus monoclonal antibodies.", ANTIMICROBIAL AGENTS AND CHEMOTHERAPY APR 2007 LNKD- PUBMED:17242138, vol. 51, no. 4, April 2007 (2007-04), pages 1386-1397, ISSN: 0066-4804
- "Fundamental Immunology: chapter 8 - Passage ED - William E Paul", FUNDAMENTAL IMMUNOLOGY,, 1 January 1993 (1993-01-01), page 242, XP008136303, ISBN: 0-7817-0022-1

## Description

The present invention relates to antibodies against CCR5, methods for their production, pharmaceutical compositions containing said antibodies, and uses thereof.

Over the past few years a growing understanding of the specific mechanisms that HIV-1 uses to enter target cells has emerged. This facilitated efforts to develop drugs that attack discrete steps in this process. The first drug targeting the entry has recently been approved for clinical use (enfuvirtide, T20; Lazzarin, A., et al., N. Engl. J. Med. 348 (2003) 2186-2195). Enfuvirtide is a peptide drug that blocks fusion at a stage subsequent to chemokine receptor binding.

HIV-1 infection is initiated by interactions between the viral envelope glycoprotein (Env) and a cellular receptor complex comprised of CD4 plus a chemokine receptor (Pierson, T.C. and Doms, R.W., Immuno. Lett. 85 (2003) 113-118; Kilby, J.M. and Eron, J.J., N. Engl. J. Med. 348 (2003) 2228-2238). Env has two subunits: the surface glycoprotein gp 120 which interacts with the cellular CD4-receptor and which is non-covalently associated with the virus transmembrane subunit gp 41. Gp 41 anchors gp 120 to the viral membrane and is also responsible for fusion. Binding of gp 120 to CD4 on cells triggers a conformational change that exposes or creates a binding site that enables gp 120 to interact with a cell surface chemokine receptor, the "co-receptor". Chemokine receptors are seven transmembrane G-protein coupled receptors (7 TM GPCRs) that normally transmit signals in response to chemokines, small cytokines with chemotactic, inflammatory and other functions.

A large proportion of drugs in clinical use are directed at other 7 TM GPCRs, and so targeting these molecules to block viral entry is an extension of the most successful type of drug development programs of the past. HIV-1 isolates require CD4 and a coreceptor to enter and infect cells. The human CC chemokine receptor CCR5 is a co-receptor for macrophage-tropic (R5) strains and plays a crucial role in the sexual transmission of HIV-1 (Berger, E.A., AIDS 11 (Suppl. A) (1997) 3-16; Bieniasz, P.D. and Cullen, B.R., Frontiers in Bioscience 3 (1998) d44-d58; Littman, D.R., Cell 93 (1998) 677-680).

Human CCR5 (further nominated as "CCR5") is used by most HIV-1 primary isolates and is critical for the establishment and maintenance of infection. In addition CCR5 function is dispensable for human health. A mutant CCR5 allele, "CCR5 Δ 32", encodes a truncated, non-functional protein (Samson, M., et al., Nature 382 (1996) 722-725; Dean, M., et al., Science 273 (1996) 1856-1862). Individuals homozygous for the mutation lack CCR5 expression and are strongly protected from HIV-1 infection. They demonstrate no overt phenotype consequence and are highly resistant to M tropic HIV infection, whereas heterozygote individuals present delayed disease progression (Schwarz, M.K. and Wells, T.N., Nat. Rev. Drug Discov. 1 (2002) 347-358). The lack of CCR5 is without apparent adverse consequences, probably because CCR5 is part of a highly redundant chemokine network as receptor for the α chemokines MIP-1α, MIP-1ß, and RANTES, which share many overlapping functions, and most of which have alternative receptors (Rossi, D. and Zlotnik, A., Annu. Rev. Immunol. 18 (2000) 217-242). The identification of CCR5 as an HIV-1 co-receptor was based on the ability of its ligands, MIP-1α, MIP-1ß, and RANTES to block infection by R5 but not R5X4 or X4 isolates (Cocchi, F., et al., Science 270 (1995) 1811-1815).

CCR5 is also a receptor of the "cluster" chemokines that are produced primarily during inflammatory responses and control the recruitment of neutrophils, macrophages, and subsets of T cells (T helper Th1 and Th2 cells). Th1 responses are typically those involving cell-mediated immunity effective against viruses and tumors, for example, whereas Th2 responses are believed to be pivotal in allergies. Therefore, inhibitors of these chemokine receptors may be useful as immunomodulators. For Th1 responses, overactive responses are dampened, for example, in autoimmunity including rheumatoid arthritis or, for Th2 responses, asthma attacks or allergic responses including atopic dermatitis are lessened (see e.g. Schols, D., Curr. Top. Med. Chem. 4 (2004) 883-893; Mueller, A. and Strange, P.G., Int. J. Biochem. Cell Biol. 36 (2004) 35-38; Kazmierski, W.M., et al., Curr. Drug Targets Infect. Disord. 2 (2002) 265-278; Lehner, T., Trends Immunol. 23 (2002) 347-351).

Antibodies against CCR5 are e.g. PRO 140 (Olson, W.C., et al., J. Virol. 73 (1999) 4145-4155) and 2D7 (Samson, M., et al., J. Biol. Chem. 272 (1997) 24934-24941). Additional antibodies are mentioned in WO 2006/103100, US 2004/0043033, US 6,610,834, US 2003/0228306, US 2003/0195348, US 2003/0166870, US 2003/0166024, US 2003/0165988, US 2003/0152913, US 2003/0100058, US 2003/0099645, US 2003/0049251, US 2003/0044411, US 2003/0003440, US 6,528,625, US 2002/0147147, US 2002/0146415, US 2002/0106374, US 2002/0061834, US 2002/0048786, US 2001/0000241, EP 1 322 332, EP 1263 791, EP 1 207 202, EP 1 161 456, EP 1 144 006, WO 2003/072766, WO 2003/066830, WO 2003/033666, WO 2002/083172, WO 02/22077, WO 01/58916, WO 01/58915, WO 01/43779, WO 01/42308.

Olson, W.C., et al. (J. Virol. 73 (1999) 4145-4155) report differential inhibition of human immunodeficiency virus type 1 fusion, gp120 binding, and CC-chemokine activity by monoclonal antibodies to CCR5. Anti-CCR5 antibodies and kits comprising same are reported in US 6,528,625. In WO 2005/105841 human G-protein chemokine receptor (CCR5) HDGNR10 is reported. The generation and characterization of a recombinant human CCR5-specific antibody is reported by Steinberger, P., et al. (J. Biol. Chem. 275 (2000) 36073-36078.

The object of the invention is to provide novel antibodies against CCR5 which are primarily used as a therapeutic agent for AIDS.

### Summary of the Invention

The invention comprises an antibody binding to CCR5, characterized in that the heavy chain variable domain comprises an amino acid sequence of the formula Gln-Val-Gln-Leu-X01-X02-Ser-Gly-Pro-Gly-Leu-Val-X03-Pro-Ser-Gln-Ser-Leu-Ser-Ile-Thr-Cys-Thr-Val-Ser-Gly-Phe-Pro-Leu-Gly-Ala-Phe-Gly-Val-His-Trp-Val-Arg-Gln-Ser-Pro-Gly-Lys-Gly-X04-Glu-Trp-Leu-Gly-Val-Ile-Trp-Lys-Gly-Gly-Asn-Thr-Asp-Tyr-Asn-Ala-Ala-Phe-X05-Ser-Arg-Leu-Arg-Ile-Thr-Lys-Asp-Asn-Ser-Lys-Ser-Gln-Val-Phe-Phe-Arg-Met-Asn-Ser-Leu-Gln-Thr-Asp-Asp-Thr-Ala-X06-Tyr-Tyr-Cys-Ala-Lys-Val-Asn-Leu-Ala-Asp-Ala-Met-Asp-Tyr-Trp-Gly-Gln-Gly-Thr-X07-Val-X08-Val-Ser-Ser,
wherein
X01 is Gln,
X02 is Glu,
X03 is Lys,
X04 is Leu or Pro,
X05 is Lys,
X06 is Ile or Thr,
X07 is Thr,
X08 is Thr
(SEQ ID NO:1), and
characterized in that the light chain variable domain of said antibody comprises an amino acid sequence of the formula
Asp-Ile-Gln-Met-Thr-Gln-Ser-Pro-Ala-Ser-Leu-Ser-Ala-Ser-Val-Gly-Glu-Thr-Val-Thr-Ile-Thr-Cys-Arg-Ala-Ser-Gly-Asn-X10-His-Gly-Tyr-Leu-Ala-Trp-X11-Gln-Gln-Lys-X12-Gly-Lys-X13-Pro-X14-Leu-Leu-X15-Tyr-Asn-Thr-Lys-Thr-Leu-Ala-Glu-Gly-Val-Pro-Ser-Arg-Phe-Ser-Gly-Ser-Gly-Ser-Gly-Thr-X16-Phe-X17-X18-X19-Ile-X20-Ser-X21-Gln-Pro-Glu-Asp-Phe-X22-X23-Tyr-Tyr-Cys-Gln-His-His-Tyr-Asp-Leu-Pro-Arg-Thr-Phe-Gly-Gly-Gly-Thr-Lys-X24-Glu-Ile-Lys,
wherein
X10 is Ala,
X11 is Tyr,
X12 is Pro,
X13 is Ala,
X14 is Lys,
X15 is Val or Ile,
X16 is Gln,
X17 is Ser,
X18 is Leu,
X19 is Lys,
X20 is Asn,
X21 is Ala,
X22 is Gly,
X23 is Asn,
X24 is Val
(SEQ ID NO: 2).

Preferably the antibody is characterized in being of human IgG4 isotype or of human IgG1 isotype, said IgG1 isotype is optionally modified in the hinge region at amino acid position 216-240 between C_{H}1 and C_{H}2 and/or in the second inter-domain region at amino acid position 327-331 between C_{H}2 and C_{H}3.

A preferred embodiment of the invention is a pharmaceutical composition comprising an antibody according to the invention.

A preferred embodiment of the invention is the use of an antibody according to the invention for the manufacture of a pharmaceutical composition.

A preferred embodiment of the invention is a method for the manufacture of a pharmaceutical composition comprising an antibody according to the invention. The antibody according to the invention is preferably characterized in that said antibody binds to CCR5 and comprises a variable heavy or light chain domain selected from the group of variable domains comprising heavy chain variable domains of SEQ ID NO: 6, 7, 8, light chain variable domains of SEQ ID NO: 9, 10, or a CCR5-binding fragment thereof.

The antibody according to the invention is preferably characterized in containing as heavy chain variable domain a heavy chain variable domain selected from the group of heavy chain variable domains of SEQ ID NO: 6, 7, or 8, or a CCR5-binding fragment thereof, and in containing as light chain variable domain a light chain variable domain selected from the group of light chain variable domains of SEQ ID NO: 9, or 10, or a CCR5-binding fragment thereof, wherein said heavy and light chain variable domains are selected independently of each other.

The antibody according to the invention is preferably characterized in that the heavy chain variable domain comprises an amino acid sequence selected from the group consisting of heavy chain variable domain amino acid sequences of SEQ ID NO: 6, 7, 8, to be more precise the antibody comprises an amino acid sequence selected from the heavy chain variable domains of SEQ ID NO: 6, 7, or 8, or a CCR5-binding fragment thereof.

The antibody according to the invention is preferably characterized in that the light chain variable domain comprises an amino acid sequence selected from the group consisting of light chain variable domain amino acid sequences of SEQ ID NO: 9, 10, to be more precise the antibody comprises an amino acid sequence selected from the light chain variable domains of SEQ ID NO: 9, or 10, or a CCR5-binding fragment thereof.

The antibody according to the invention is preferably characterized in that the constant regions (light and heavy chains) are of human origin. Such constant regions (chains) are well known in the state of the art and e.g. described by Kabat (see e.g. Johnson, G. and Wu, T.T., Nucleic Acids Res. 28 (2000) 214-218). For example, a useful human heavy chain constant region comprises an amino acid sequence independently selected from the group consisting of SEQ ID NO: 3, 4. For example, a useful human light chain constant region comprises an amino acid sequence of a kappa-light chain constant region of SEQ ID NO: 5. It is further preferred that the antibody is of mouse origin and comprises the antibody variable sequence frame of a mouse antibody according to Kabat (see e.g. Johnson, G. and Wu, T.T., Nucleic Acids Res. 28 (2000) 214-218).

The antibodies inhibit one or more functions of human CCR5, such as ligand binding to CCR5, signaling activity (e.g. activation of a mammalian G protein, induction of a rapid and transient increase in the concentration of cytosolic free Ca²⁺, and/or stimulation of a cellular response (e.g. stimulation of chemotaxis, exocytosis or inflammatory mediator release by leukocytes, integrin activation)). The antibodies inhibit binding of RANTES, MIP-1 alpha, MIP-1 beta, and/or HIV to human CCR5 and inhibit functions mediated by human CCR5, like leukocyte trafficking, HIV entry into a cell, T cell activation, inflammatory mediator release, and/or leukocyte degranulation.

The antibody according to the invention specifically binds to human CCR5 and inhibits HIV fusion with a target cell in an assay comprising contacting the said target cells with the antibody in the presence of the virus with an antibody concentration effective to inhibit membrane fusion between the virus and said cell with an IC₅₀ value of 4.0 µg/ml or lower.

The antibody according to the invention specifically binds to CCR5 and inhibits membrane fusion between a first cell co-expressing CCR5 and CD4 polypeptides and a second cell expressing an HIV env protein with an IC₅₀ value of 1.5 µg/ml or lower, preferably 0.3 µg/ml or lower.

The antibody according to the invention specifically binds to CCR5 and inhibits stimulation of a cellular response in a target cell, preferably inhibits migration, in an assay comprising contacting said target cell with the antibody in the presence of RANTES, MIP-1 alpha, and/or MIP-1 beta with an IC₅₀ value of 1.5 µg/ml or lower.

An antibody according to the invention preferably does not inhibit chemokine binding in a binding assay to CCR1, CCR2, CCR3, CCR4, CCR6, and CXCR4 in an antibody concentration up to 100 µg/ml.

An antibody according to the invention preferably does not stimulate intracellular Ca²⁺ increase, detected in CHO cells expressing CCR5 and Galpha16 in an antibody concentration up to 50 µg/ml.

The antibody according to the invention is preferably of human isotype IgG1, IgG2, IgG3, or IgG4, whereby IgG1 or IgG4 are preferred.

The antibody according to the invention is preferably of IgG4 isotype. The antibody according to the invention is preferably of IgG1 isotype. The antibody according to the invention is preferably of IgG4 isotype with mutation S228P. The antibody according to the invention, i.e. the heavy and light chain constant region, is of IgG1 or IgG4 isotype modified in the hinge region at about amino acid position 216-240, preferably at about amino acid position 220-240, between C_{H}1 and C_{H}2 (Angal, S., et al., Mol. Immunol. 30 (1993) 105-108), and/or in the second inter-domain region at about amino acid position 327-331 between C_{H}2 and C_{H}3 (numbering according to Kabat, see e.g. Johnson, G. and Wu, T.T., Nucleic Acids Res. 28 (2000) 214-218). Such modifications reduce or avoid effector function (ADCC and/or CDC). Switching of IgG class can be performed by exchange of the heavy chain constant region and light chain constant domain of the antibody by those from an antibody of the desired class, like IgG 1 mutants or IgG4. Such methods are well known in the state of the art.

The antibody according to the invention is preferably characterized by being of human subclass IgG1, containing at least one mutation in L234 (leucine at amino acid position 234), L235, D270, N297, E318, K320, K322, P331, and/or P329 (numbering according to EU index). Preferably the antibody is of human IgG1 isotype comprising mutations L234A (alanine instead of leucine at amino acid position 234) and L235A. The antibody according to the invention is preferably characterized by being of human IgG4 isotype containing a mutation at position S228.

The invention relates therefore in one aspect to antibodies, characterized in that said antibodies bind CCR5, contain an Fc part from human origin, and do not bind human complement factor C1q and/or activate complement factor C3. Preferably the antibodies show a reduced binding to or do not bind to human Fcγ receptor.

The invention further comprises a nucleic acid molecule encoding an antibody binding to CCR5 according to the invention.

The invention further provides expression vectors containing said nucleic acid according to the invention capable of expressing said nucleic acid in a prokaryotic or eukaryotic host cell, and host cells containing such vectors for the recombinant production of such an antibody.

The invention further comprises a prokaryotic or eukaryotic host cell comprising a vector according to the invention.

The invention further comprises a method for the production of a recombinant human or humanized antibody according to the invention, characterized by expressing a nucleic acid according to the invention in a prokaryotic or eukaryotic host cell and recovering said antibody from said cell or the cell culture supernatant. The invention further comprises the antibody obtainable by such a recombinant method.

Antibodies according to the invention show benefits for patients in need of a CCR5 targeting therapy. The antibodies according to the invention have new and inventive properties causing a benefit for a patient suffering from such a disease, especially suffering from immunosuppression, especially suffering from HIV infection.

The antibody is administered preferably in a pharmaceutical composition.

The invention further comprises the use of an antibody according to the invention as a medicament, for the treatment of an immunosuppressive disease, preferably for the treatment of HIV infection, for the treatment of a patient suffering from immunosuppression, and for the manufacture of a pharmaceutical composition according to the invention. In addition, the invention comprises a method for the manufacture of a pharmaceutical composition according to the invention.

The invention further comprises a pharmaceutical composition containing an antibody according to the invention in a pharmaceutically effective amount, optionally together with a buffer and/or an adjuvant useful for the formulation of antibodies for pharmaceutical purposes.

The invention further provides pharmaceutical compositions comprising such antibodies in a pharmaceutically acceptable carrier. In one embodiment, the pharmaceutical composition may be included in an article of manufacture or kit.

Therefore one aspect of the current invention is an antibody according to the invention for use as a medicament. Another aspect of the invention is an antibody according to the invention for use for the treatment of an immunosuppressive disease. Also an aspect is the use of an antibody according to the invention for the manufacture of a medicament for the treatment of an immunosuppressive disease

### Detailed Description of the Invention

The term "antibody" encompasses the various forms of antibody structures including but not being limited to whole antibodies, and antibody fragments. The antibody according to the invention is preferably a humanized antibody, chimeric antibody, or further genetically engineered antibody as long as the characteristic properties according to the invention are retained.

"Antibody fragments" comprise a portion of a full length antibody, preferably the variable domain thereof, or at least the antigen binding site thereof. Examples of antibody fragments include diabodies, single-chain antibody molecules, immunotoxins, and multispecific antibodies formed from antibody fragments. scFv antibodies are, e.g., described in Huston, J.S., Methods in Enzymol. 203 (1991) 46-88. In addition, antibody fragments comprise single chain polypeptides having the characteristics of a V_{H} domain, namely being able to assemble together with a V_{L} domain, or of a V_{L} domain binding to CCR5, namely being able to assemble together with a V_{H} domain to a functional antigen binding site and thereby providing the property of inhibiting membrane fusion or HIV fusion with a target cell.

The terms "monoclonal antibody" or "monoclonal antibody composition" as used herein refer to a preparation of antibody molecules of a single amino acid composition. The term "chimeric antibody" refers to a monoclonal antibody comprising a variable domain, i.e. binding region, from mouse and at least a portion of a constant region derived from a different source or species, usually prepared by recombinant DNA techniques. Chimeric antibodies comprising a mouse variable domain and a human constant region are especially preferred. Such mouse/human chimeric antibodies are the product of expressed immunoglobulin genes comprising DNA segments encoding mouse immunoglobulin variable domains and DNA segments encoding human immunoglobulin constant regions. Other forms of "chimeric antibodies" encompassed by the present invention are those in which the class or subclass has been modified or changed from that of the original antibody. Such "chimeric" antibodies are also referred to as "class-switched antibodies." Methods for producing chimeric antibodies involve conventional recombinant DNA and gene transfection techniques well known in the art. See, e.g., Morrison, S.L., et al., Proc. Natl. Acad. Sci. USA 81 (1984) 6851-6855; US Patent Nos. 5,202,238 and 5,204,244.

The term "humanized antibody" refers to antibodies in which the framework and/or "complementarity determining regions" (CDR) have been modified to comprise the CDR of an immunoglobulin of different species as compared to that of the parent immunoglobulin. In a preferred embodiment, a mouse CDR is grafted into the framework region of a human antibody to prepare the "humanized antibody". See, e.g., Riechmann, L., et al., Nature 332 (1988) 323-327; and Neuberger, M.S., et al., Nature 314 (1985) 268-270. Particularly preferred CDRs correspond to those representing sequences recognizing the antigens noted above for chimeric and bifunctional antibodies.

The term "binding to CCR5" as used herein means binding of the antibody to CCR5 in a cell based in vitro ELISA assay (CCR5 expressing cells, e.g. transformed CHO cells, L1.2 cells). Binding is found if the antibody causes an S/N (signal/noise) ratio of 5 or more, preferably of 10 or more, at an antibody concentration of 100 ng/ml.

The term "seven transmembrane chemokine molecular structure" as used herein refers to the natural structure CCR5 shows when it is positioned in the cell membrane bilayer (see, e.g., Oppermann, M., Cell. Sig. 16 (2004) 1201-1210). Like other G protein-coupled receptors (e.g. G protein-coupled receptor 1b), CCR5 is composed of an extracellular N-terminal domain, a transmembrane domain and a cytoplasmatic C-terminal domain. The transmembrane domain consists of seven hydrophobic transmembrane segments, linked by three cytoplasmatic and three extracellular segments. The antibody according to the invention binds to CCR5 in its seven transmembrane chemokine molecular structure.

The term "epitope" denotes a protein determinant capable of specifically binding to an antibody. Epitopes usually consist of chemically active surface groupings of molecules such as amino acids or sugar side chains and usually epitopes have specific three dimensional structural characteristics, as well as specific charge characteristics. Conformational and non-conformational epitopes are distinguished in that the binding to the former but not the latter is lost in the presence of denaturing solvents. Preferably an antibody according to the invention binds specifically to native but not to denatured CCR5.

The term "membrane fusion" refers to fusion between a first cell coexpressing CCR5 and CD4 polypeptides and a second cell expressing an HIV env protein. Membrane fusion is determined by luciferase reporter gene assay.

The term "inhibiting HIV fusion with a target cell" refers to inhibiting HIV fusion with a target cell measured in an assay comprising contacting the target cell with the antibody in the presence of said virus with an antibody concentration effective to inhibit membrane fusion between the virus and said cell, and measuring luciferase reporter gene activity.

The "variable domain" (variable domain of a light chain (V_{L}), variable domain of a heavy chain (V_{H})) as used herein denotes each of the pair of light and heavy chain domains which are involved directly in binding the antibody to the antigen. The variable light and heavy chain domains have the same general structure and each domain comprises four framework regions (FR), whose sequences are widely conserved, connected by three "hypervariable regions" (or complementarity determining regions, CDRs). The framework regions adopt a β-sheet conformation and the CDRs may form loops connecting the β-sheet structure. The CDRs in each chain are held in their three-dimensional structure by the framework regions and form together with the CDRs from the other chain the antigen binding site. The antibody's heavy and light chain CDR3 regions play a particularly important role in the binding specificity/affinity of the antibodies according to the invention and therefore provide a further object of the invention.

The antibody according to the invention is preferably characterized in that said antibody comprises a heavy chain variable domain and a light chain variable domain selected from the group of combinations consisting of
a) the heavy chain variable domain defined by amino acid sequence of SEQ ID NO: 6 and the light chain variable domain defined by amino acid sequence of SEQ ID NO: 9;
b) the heavy chain variable domain defined by amino acid sequence of SEQ ID NO: 6 and the light chain variable domain defined by amino acid sequence of SEQ ID NO: 10;
c) the heavy chain variable domain defined by amino acid sequence of SEQ ID NO: 7 and the light chain variable domain defined by amino acid sequence of SEQ ID NO: 9;
d) the heavy chain variable domain defined by amino acid sequence of SEQ ID NO: 7 and the light chain variable domain defined by amino acid sequence of SEQ ID NO: 10;
e) the heavy chain variable domain defined by amino acid sequence of SEQ ID NO: 8 and the light chain variable domain defined by amino acid sequence of SEQ ID NO: 9;
f) the heavy chain variable domain defined by amino acid sequence of SEQ ID NO: 8 and the light chain variable domain defined by amino acid sequence of SEQ ID NO: 10.

The term "antigen-binding portion of an antibody" when used herein refer to the amino acid residues of an antibody which are responsible for antigen-binding. The antigen-binding portion of an antibody comprises amino acid residues from the "complementarity determining regions" or "CDRs". "Framework" or "FR" regions are those variable domain regions other than the hypervariable region residues as herein defined. Therefore, the light and heavy chain variable domains of an antibody comprise from N- to C-terminus the domains FR1, CDR1, FR2, CDR2, FR3, CDR3, and FR4. Especially, CDR3 of the heavy chain is the region which contributes most to antigen binding and defines the antibody's properties. CDR and FR regions are determined according to the standard definition of Kabat et al., Sequences of Proteins of Immunological Interest, 5th ed., Public Health Service, National Institutes of Health, Bethesda, MD, Publication No. 91-3242 (1991) and/or those residues from a "hypervariable loop".

The terms "nucleic acid" or "nucleic acid molecule", as used herein, are intended to include DNA molecules and RNA molecules. A nucleic acid molecule may be single-stranded or double-stranded, but preferably is double-stranded DNA.

The term "amino acid" as used within this application denotes the group of naturally occurring carboxy α-amino acids comprising alanine (three letter code: ala, one letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

A nucleic acid is "operably linked" when it is placed into a functional relationship with another nucleic acid. For example, DNA for a pre-sequence or secretory leader is operably linked to DNA for a polypeptide if it is expressed as a pre-protein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are co-linear, and, in the case of a secretory leader, contiguous and in reading frame. However, enhancers do not have to be contiguous. Linking is accomplished by ligation at convenient restriction sites. If such sites do not exist, synthetic oligonucleotide adaptors or linkers are used in accordance with conventional practice.

As used herein, the expressions "cell", "cell line", and "cell culture" are used interchangeably and all such designations include progeny. Thus, the words "transformants" and "transformed cells" include the primary subject cell and cultures derived there from without regard for the number of transfers. It is also understood that all progeny may not be precisely identical in DNA content, due to deliberate or inadvertent mutations. Variant progeny that have the same function or biological activity as screened for in the originally transformed cell are included.

The "Fc part" of an antibody is not involved directly in binding of an antibody to an antigen, but exhibit various effector functions. Depending on the amino acid sequence of the constant region of their heavy chains, antibodies or immunoglobulins are divided in the classes: IgA, IgD, IgE, IgG, and IgM, and several of these may be further divided into subclasses (isotypes), e.g. IgG in IgG1, IgG2, IgG3, and IgG4, IgA in IgA1, and IgA2. According to the heavy chain constant regions the different classes of immunoglobulins are called α, δ, ε, γ, and µ, respectively. The antibodies according to the invention are preferably of IgG type.

As used herein the term "Fc part derived from human origin" denotes an Fc part which is either an Fc part of a human antibody of the subclass IgG4 or an Fc part of a human antibody of the subclass IgG1, IgG2, or IgG3, including mutated forms thereof. Preferably the Fc part of a human antibody of the subclass IgG1, IgG2, or IgG3 is modified in such a way that a reduced or no Fcγ receptor (FcγR, i.e. FcγRIIIa) binding and/or a reduced or no C1q binding as defined below can be detected with respect to the non-modified Fc part. An "Fc part of an antibody" is a term well known to the skilled artisan and defined on the basis of papain cleavage of antibodies. The antibodies according to the invention contain as Fc part an Fc part derived from human origin and preferably all other parts of the human constant region. Preferably the Fc part is a human Fc part and especially preferred either from human IgG4 subclass, or from human IgG1 subclass, or a mutated Fc part from human IgG2 subclass. Mostly preferred are the Fc parts and heavy chain constant region shown in SEQ ID NO: 3, SEQ ID NO: 4, SEQ ID NO: 3 with mutations L234A and L235A, SEQ ID NO: 4 with mutation S228P.

While IgG4 shows reduced Fcγ receptor (FcγRIIIa) binding, antibodies of other IgG subclasses show strong binding. However Pro238, Asp265, Asp270, Asn297 (loss of Fc carbohydrate), Pro329, Leu234, Leu235, Gly236, Gly237, Ile253, Ser254, Lys288, Thr307, Gln311, Asn434, and His435 are residues which, if altered, provide also reduced Fc receptor binding (Shields, R.L., et al., J. Biol. Chem. 276 (2001) 6591-6604; Lund, J., et al., FASEB J. 9 (1995) 115-119; Morgan, A., et al., Immunology 86 (1995) 319-324; EP 0 307 434). Preferably an antibody according to the invention is in regard to Fcγ receptor binding of IgG4 subclass, or of IgG1 or IgG2 subclass, with a mutation in S228, L234, L235, and/or D265, and/or contains the PVA236 mutation. Preferred are the mutations S228P, L234A, L235A, L235E, and/or PVA236 (PVA236 means that the amino acid sequence ELLG (given in one letter amino acid code) from amino acid position 233 to 236 of IgG1 or EFLG of IgG4 is replaced by PVA). Especially preferred are the mutations S228P of IgG4, and L234A, L235A of IgG1.

The Fc part of an antibody is directly involved in ADCC (antibody-dependent cell-mediated cytotoxicity) and CDC (complement-dependent cytotoxicity). Complement activation (CDC) is initiated by binding of complement factor C1q to the Fc part of most IgG antibody subclasses. Binding of C1q to an antibody is caused by defined protein-protein interactions at the so called binding site. Such Fc part binding sites are known in the state of the art and described e.g. by Lukas, T.J., et al., J. Immunol. 127 (1981) 2555-2560; Brunhouse, R. and Cebra, J.J., Mol. Immunol. 16 (1979) 907-917; Burton, D.R., et al., Nature 288 (1980) 338-344; Thommesen, J.E., et al., Mol. Immunol. 37 (2000) 995-1004; Idusogie, E.E., et al., J. Immunol. 164 (2000) 4178-4184; Hezareh, M., et al., J. Virol. 75 (2001) 12161-12168; Morgan, A., et al., Immunology 86 (1995) 319-324; and EP 0 307 434. Such Fc part binding sites are, e.g., characterized by the amino acids L234, L235, D270, N297, E318, K320, K322, P331, and P329 (numbering according to EU index of Kabat). Antibodies of subclass IgG1, IgG2, and IgG3 usually show complement activation including C1q and C3 binding, whereas IgG4 does not activate the complement system and does not bind C1q and C3.

That is, in cases in which ADCC and/or CDC is/are required, an Fc part of IgG1 subclass is preferred, in cases in which reduced or no ADCC and/or CDC is/are required, an Fc part of IgG4 subclass, or modified/mutated IgG1 subclass is preferred. The present invention refers in one aspect to an antibody that binds CCR5 and shows reduced binding to or does not bind Fcγ receptor and/or complement factor C1q. An anti-CCR5 antibody which does not bind Fc receptor and/or complement factor C1q does not elicit antibody-dependent cellular cytotoxicity (ADCC) and/or complement dependent cytotoxicity (CDC), whereas an anti-CCR5 antibody, which shows reduced binding to Fc receptor and/or complement factor C1q, shows a reduced ADCC and/or CDC. Preferably, such an antibody is characterized in that it binds CCR5, contains an Fc part derived from human origin, and does not bind or shows a reduced binding of Fc receptors and/or complement factor C1q. More preferably, this antibody is a human or humanized antibody or a T-cell antigen depleted antibody. C1q binding can be measured according to Idusogie, E.E., et al., J. Immunol. 164 (2000) 4178-4184. No "C1q binding" is found if in such an assay the optical density (OD) at 492-405 nm is for the test antibody lower than 15% of the value for human C1q binding of the unmodified wild-type antibody Fc part at an antibody concentration of 8 µg/ml. Reduced "C1q binding" is in the range of from 15 % to 30 % of the value for human C1q binding of the unmodified wild-type antibody Fc part at the same conditions. ADCC can be measured as binding of the antibody to human FcγRIIIa on human NK cells. Binding is determined at an antibody concentration of 20 µg/ml. "No Fcγ receptor binding" or "no ADCC" means a binding of up to 30% to human FcγRIIIa on human NK cells at an antibody concentration of 20 µg/ml compared to the binding of the same antibody as human IgG1 (SEQ ID NO: 3). "Reduced Fcγ receptor binding" or "reduced ADCC" means a binding of from 30 % up to 60 % to human FcγRIIIa on human NK cells compared to the binding of the same antibody as human IgG1 (SEQ ID NO: 3).

Another aspect of the current invention is an antibody that binds CCR5 and also does bind Fcγ receptor and/or complement factor C1q. An anti-CCR5 antibody which does bind Fc receptor and/or complement factor C1q does elicit antibody-dependent cellular cytotoxicity (ADCC) and/or complement dependent cytotoxicity (CDC). Preferably, this antibody is characterized in that it binds CCR5, contains an Fc part derived from human origin, and does also bind Fc receptors and/or complement factor C1q. More preferably, this antibody is a human or humanized antibody or a T-cell antigen depleted antibody. C1q binding can be measured according to Idusogie, E.E., et al., J. Immunol. 164 (2000) 4178-4184. ADCC can be measured as binding of the antibody to human FcγRIIIa on human NK cells. Binding is determined at an antibody concentration of 20 µg/ml.

The antibodies according to the invention include, in addition, such antibodies having "conservative sequence modifications" (variant antibodies), nucleotide and amino acid sequence modifications, which do not affect or alter the abovementioned characteristics of the antibody according to the invention. Modifications can be introduced by standard techniques known in the art, such as site-directed mutagenesis and PCR-mediated mutagenesis. Conservative amino acid substitutions include ones in which the amino acid residue is replaced with an amino acid residue having a similar side chain. Families of amino acid residues having similar side chains have been defined in the art. These families include amino acids with basic side chains (e.g. lysine, arginine, histidine), acidic side chains (e.g. aspartic acid, glutamic acid), uncharged polar side chains (e.g. glycine, asparagine, glutamine, serine, threonine, tyrosine, cysteine, tryptophan), non-polar side chains (e.g. alanine, valine, leucine, isoleucine, proline, phenylalanine, methionine), beta-branched side chains (e.g. threonine, valine, isoleucine) and aromatic side chains (e.g. tyrosine, phenylalanine, tryptophan, histidine). Thus, a predicted nonessential amino acid residue in a human anti-CCR5 antibody can be preferably replaced with another amino acid residue from the same side chain family. A "variant" anti-CCR5 antibody, refers therefore herein to a molecule which differs in amino acid sequence from a "parent" anti-CCR5 antibody's amino acid sequence by up to ten, preferably from about two to about five, additions, deletions and/or substitutions in one or more variable region of the parent antibody. Amino acid substitutions can be performed by mutagenesis based upon molecular modeling as described by Riechmann, L., et al., Nature 332 (1988) 323-327 and Queen, C., et al., Proc. Natl. Acad. Sci. USA 86 (1989) 10029-10033.

A further embodiment of the invention is a method for the production of an antibody against CCR5 which does not bind or shows a reduced binding to Fcγ receptor and/or C1q, characterized in that the sequence of a nucleic acid encoding the heavy chain of a human IgG1 type antibody binding to CCR5 is modified in such a manner that said modified antibody does not bind or shows a reduced binding of C1q and/or Fcγ receptor, said modified nucleic acid and the nucleic acid encoding the light chain of said antibody are inserted into an expression vector, said vector is inserted in a eukaryotic host cell, the encoded protein is expressed and recovered from the host cell or the supernant. Preferably the antibody is modified by "class switching", i.e. change or mutation of the Fc part (e.g. from IgG1 to IgG4, and/or IgG1/IgG4 mutation) preferably defined as IgG1v1 (PVA-236; GLPSS331), IgG1v2 (L234A; L235A), IgGlv3 (S228P; L235E), IgGlx (S228P), IgG4v1 (PVA-236). GLPSS331 means the mutations E233P, L234V, L235A, delta G236, A327G, A330S, P331S.

Identity or homology with respect to the sequence is defined herein as the percentage of amino acid residues in the candidate sequence that are identical with the parent sequence, after aligning the sequences and introducing gaps, if necessary, to achieve the maximum percent sequence identity. None of N-terminal, C-terminal, or internal extensions, deletions, or insertions into the antibody sequence shall be construed as affecting sequence identity or homology. The variant retains the ability to bind human CCR5 and preferably has properties, which are superior to those of the parent antibody. For example, the variant may have reduced side effects during treatment.

The "parent" antibody herein is one, which is encoded by an amino acid sequence used for the preparation of the variant. Preferably, the parent antibody has a human framework region and, if present, has a human antibody constant region or human antibody constant domains. For example, the parent antibody may be a humanized or a human antibody.

The antibodies according to the invention are preferably produced by recombinant means. Such methods are widely known in the state of the art and comprise protein expression in prokaryotic and eukaryotic cells with subsequent isolation of the antibody polypeptide and usually purification to a pharmaceutically acceptable purity. For the protein expression nucleic acids encoding light and heavy chains or fragments thereof are inserted into expression vectors by standard methods. Expression is performed in appropriate prokaryotic or eukaryotic host cells, such as CHO cells, BHK cells, PER.C6^{®} cells, NSO cells, SP2/0 cells, HEK293 cells, COS cells, yeast, or E. coli cells, and the antibody is recovered from the cells (from the supernatant or after cells lysis).

Recombinant production of antibodies is well-known in the state of the art and described, for example, in the review articles of Makrides, S.C., Protein Expr. Purif. 17 (1999) 183-202; Geisse, S., et al., Protein Expr. Purif. 8 (1996) 271-282; Kaufman, R.J., Mol. Biotechnol. 16 (2000) 151-160; Werner, R.G., Arzneimittelforschung-Drug Res. 48 (1998) 870-880.

The antibodies may be present in whole cells, in a cell lysate, or in a partially purified, or substantially pure form. Purification is performed in order to eliminate other cellular components or other contaminants, e.g. other cellular nucleic acids or proteins, by standard techniques, including alkaline/SDS treatment, CsCl banding, column chromatography, agarose gel electrophoresis, and others well known in the art. See Ausubel, F., et al., (ed.) Current Protocols in Molecular Biology, Greene Publishing and Wiley Interscience, New York (1987).

Expression in NSO cells is described by, e.g., Barnes, L.M., et al., Cytotechnology 32 (2000) 109-123; Barnes, L.M., et al., Biotech. Bioeng. 73 (2001) 261-270. Transient expression is described by, e.g., Durocher, Y., et al., Nucl. Acids. Res. 30 (2002) E9. Cloning of variable domains is described by Orlandi, R., et al., Proc. Natl. Acad. Sci. USA 86 (1989) 3833-3837; Carter, P., et al., Proc. Natl. Acad. Sci. USA 89 (1992) 4285-4289; Norderhaug, L., et al., J. Immunol. Methods 204 (1997) 77-87. A preferred transient expression system (HEK 293) is described by Schlaeger, E.-J. and Christensen, K., in Cytotechnology 30 (1999) 71-83, and by Schlaeger, E.-J., in J. Immunol. Methods 194 (1996) 191-199.

Monoclonal antibodies are suitably separated from the culture medium by conventional immunoglobulin purification procedures such as, for example, protein A-Sepharose, hydroxyapatite chromatography, gel electrophoresis, dialysis, or affinity chromatography. DNA and RNA encoding the monoclonal antibodies is readily isolated and sequenced using conventional procedures. The hybridoma cells can serve as a source of such DNA and RNA. Once isolated, the DNA may be inserted into expression vectors, which are then transfected into host cells, such as HEK 293 cells, CHO cells, or myeloma cells that do not otherwise produce immunoglobulin protein, to obtain the synthesis of recombinant monoclonal antibodies in the host cells.

Amino acid sequence variants of human CCR5 antibody are prepared by introducing appropriate nucleotide changes into the antibody encoding DNA, or by peptide synthesis. Such modifications can be performed, however, only in a very limited range, e.g. as described above. For example, the modifications do not alter the abovementioned antibody characteristics such as the IgG isotype and epitope binding, but may improve the yield of the recombinant production, protein stability, or facilitate the purification.

Any cysteine residue not involved in maintaining the proper conformation of the anti-CCR5 antibody may also be substituted, generally with serine, to improve the oxidative stability of the molecule and to prevent aberrant crosslinking. Conversely, cysteine bond(s) may be added to the antibody to improve its stability (particularly where the antibody is an antibody fragment such as an Fv fragment).

Another type of amino acid variant of the antibody alters the original glycosylation pattern of the antibody. By "altering" is meant removing one or more carbohydrate moieties found in the antibody and/or adding one or more glycosylation sites that are not present in the antibody. Glycosylation of antibodies is typically N-linked. Te term "N-linked" refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. Addition of glycosylation sites to the antibody is conveniently accomplished by altering the amino acid sequence such that it contains one or more of the above-described tripeptide sequences (for N-linked glycosylation sites).

Nucleic acid molecules encoding amino acid sequence variants of anti-CCR5 antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of humanized anti-CCR5 antibody.

Another type of covalent modification involves chemically or enzymatically coupling glycosides to the antibody. These procedures are advantageous in that they do not require production of the antibody in a host cell that is capable of N- or O-linked glycosylation. Depending on the coupling mode used, the sugar(s) may be attached to (a) arginine and/or histidine, (b) free carboxyl groups, (c) free sulfhydryl groups such as those of cysteine, (d) free hydroxyl groups such as those of serine, threonine, or hydroxyproline, (e) aromatic residues such as those of phenylalanine, tyrosine, or tryptophan, or (f) the amide group of glutamine. These methods are described in WO 87/05330, and in Aplin, J.D. and Wriston, J.C. Jr., CRC Crit. Rev. Biochem. 10 (1981) 259-306.

Removal of any carbohydrate moieties present on the antibody may be accomplished chemically or enzymatically. Chemical deglycosylation requires exposure of the antibody to the compound trifluoro methanesulfonic acid, or an equivalent compound. This treatment results in the cleavage of most or all sugars except the linking sugar (N-acetylglucosamine or N-acetyl galactosamine), while leaving the antibody intact. Chemical deglycosylation is described by Sojar, H.T. and Bahl, O.P., Arch. Biochem. Biophys. 259 (1987) 52-57; Edge, A.S., et al. Anal. Biochem. 118 (1981) 131-137. Enzymatic cleavage of carbohydrate moieties on antibodies can be achieved by the use of a variety of endo- and exoglycosidases as described by Thotakura, N.R. and Bahl, O.P., Meth. Enzymol. 138 (1987) 350-359.

Another type of covalent modification of the antibody comprises linking the antibody to one of a variety of non-proteinaceous polymers, e.g., polyethylene glycol, polypropylene glycol, or polyoxyalkylenes, in the manner set forth in US Patent Nos. 4,640,835; 4,496,689; 4,301,144; 4,670,417; 4,791,192; 4,179,337.

The heavy and light chain variable domains according to the invention are combined with sequences of promoter, translation initiation, constant region, 3' untranslated region, polyadenylation, and transcription termination to form expression vector constructs. The heavy and light chain expression constructs can be combined into a single vector, co-transfected, serially transfected, or separately transfected into host cells which are then fused to form a single host cell expressing both chains.

The invention further comprises the use of an antibody according to the invention for the diagnosis of AIDS susceptibility in vitro, preferably by an immunological assay determining the binding between soluble CCR5 of a human plasma sample (Tsimanis, T., Immunology Letters 96 (2005) 55-61) and the antibody according to the invention. Expression of CCR5 has a correlation with disease progression, and can be used to identify low or high risk individuals for AIDS susceptibility. For diagnostic purposes, the antibodies or antigen binding fragments can be labeled or unlabeled. Typically, diagnostic assays entail detecting the formation of a complex resulting from the binding of an antibody or antibody fragment to CCR5.

In another aspect, the present invention provides a composition, e.g. a pharmaceutical composition, containing one or a combination of monoclonal antibodies, or the antigen-binding portion thereof, of the present invention, formulated together with a pharmaceutically acceptable carrier.

As used herein, "pharmaceutically acceptable carrier" includes any and all solvents, dispersion media, coatings, antibacterial and antifungal agents, isotonic and absorption/resorption delaying agents, and the like that are physiologically compatible. Preferably, the carrier is suitable for injection or infusion.

A composition of the present invention can be administered by a variety of methods known in the art. As will be appreciated by the skilled artisan, the route and/or mode of administration will vary depending upon the desired results.

Pharmaceutically acceptable carriers include sterile aqueous solutions or dispersions and sterile powders for the preparation of sterile injectable solutions or dispersion. The use of such media and agents for pharmaceutically active substances is known in the art. In addition to water, the carrier can be, for example, an isotonic buffered saline solution.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient (effective amount). The selected dosage level will depend upon a variety of pharmacokinetic factors including the activity of the particular compositions of the present invention employed, or the ester, salt or amide thereof, the route of administration, the time of administration, the rate of excretion of the particular compound being employed, other drugs, compounds and/or materials used in combination with the particular compositions employed, the age, sex, weight, condition, general health and prior medical history of the patient being treated, and like factors well known in the medical arts.

The invention comprises the use of the antibodies according to the invention for the treatment of a patient suffering from immunosuppression, such as immunosuppression in a patient with immunodeficiency syndromes such as AIDS, in a patient undergoing radiation therapy, chemotherapy, therapy for autoimmune disease or other drug therapy (e.g., corticosteroid therapy), which causes immunosuppression, or for the treatment of a patient suffering from GvHD or HvGD (e.g. after transplantation). The invention comprises also a method for the treatment of a patient suffering from such immunosuppression.

The invention further provides a method for the manufacture of a pharmaceutical composition comprising an effective amount of an antibody according to the invention together with a pharmaceutically acceptable carrier and the use of the antibody according to the invention for such a method. The invention also provides an antibody according to the invention for use as a medicament. Also is provided an antibody according to the invention for the treatment of an immunosuppressive disease.

The invention further provides the use of an antibody according to the invention in an effective amount for the manufacture of a pharmaceutical agent, preferably together with a pharmaceutically acceptable carrier, for the treatment of a patient suffering from immunosuppression.

The invention also provides the use of an antibody according to the invention in an effective amount for the manufacture of a pharmaceutical agent, preferably together with a pharmaceutically acceptable carrier, for the treatment of a patient suffering from inflammatory mediator release mediated by CCR5.

The following examples and sequence listing are provided to aid the understanding of the present invention, the true scope of which is set forth in the appended claims.

### Description of the Sequences

SEQ ID NO: 1 Formula I, heavy chain, variable domain
SEQ ID NO: 2 Formula II, light chain, variable domain
SEQ ID NO: 3 γ1 heavy chain constant region
SEQ ID NO: 4 y4 heavy chain constant region
SEQ ID NO: 5 κ light chain constant region
SEQ ID NO: 6 heavy chain variable domain
SEQ ID NO: 7 heavy chain variable domain
SEQ ID NO: 8 heavy chain variable domain
SEQ ID NO: 9 light chain variable domain
SEQ ID NO: 10 light chain variable domain

### Example 1

### Recombinant production of antibodies

Vectors for the expression of antibodies according to the invention have been constructed as follows. A heavy chain expression vector was constructed by linking a heavy chain variable domain to human IgG1 (SEQ ID NO: 3) constant region in the expression vector pSVgpt. A light chain expression vector was constructed by linking a light chain variable domain to human Kappa light chain constant region (SEQ ID NO: 5) in the expression vector pSVhyg. 5' flanking sequence including the leader signal peptide, leader intron and the murine immunoglobulin promoter, and 3' flanking sequence including the splice site and intron sequence was introduced using the vectors VH-PCR1 and VK-PCR1 as templates. The heavy and light chain expression vectors were co-transfected into NS0 cells (ECACC No 85110503, a non-immunoglobulin producing mouse myeloma). Transfected cell clones were screened for production of human antibody by ELISA for human IgG.

### Example 2

### Construction of expression plasmids for mutant (variant) anti-CCR5 antibodies

Expression plasmids encoding mutant anti-CCR5 antibody heavy and light chains were created by site-directed mutagenesis of the expression plasmids using the QuickChange™ Site-Directed mutagenesis Kit (Stratagene) and are described in Table 1. Amino acids are numbered according to EU numbering (Edelman, G.M., et al., Proc. Natl. Acad. Sci. USA 63 (1969) 78-85; Kabat, E.A., et al., Sequences of Proteins of Immunological Interest, 5th ed., National Institutes of Health, Bethesda, MD, Publication No. 91-3242 (1991)).

Table 1 shows mutants of constant chains (Fc).

**Table 1:**

| **Isotype** | **Abbreviation** | **Mutations** | **Description** |
|---|---|---|---|
| IgG1 | IgG1v2 | L234A; | The amino acid sequence Leu₂₃₄Leu₂₃₅ of |
| | | L235A | the human γ1 -heavy chain is replaced by the amino acid sequence Ala₂₃₄Ala₂₃₅ |

Explanation of mutations: L234A means that leucine at Kabat amino acid position 234 is changed to alanine.

### Example 3

### Cell-Cell Fusion Assay

At day 1, gp160-expressing HeLa cells (2 x 10⁴ cells / 50µl / well) were seeded in a white 96 microtiter plate in DMEM medium supplemented with 10 % (v/v) FCS and 2 µg/ml doxycycline. At day 2, 100 µl of supernatant sample or antibody control per well was added in a dear 96 microtiter plate. Then 100 µl containing 8x10⁴ CEM-NKr-Luc suspension cells in medium were added and incubated for 30 min. at 37°C. The HeLa cell culture medium was aspirated from the 96 well plate, 100 µl from the 200 µl antibody/CEM-NKr-Luc mixture was added and incubated over night at 37 °C. At day 3, 100 µl/well Bright-Glo™ Luciferase assay substrate (1,4-dithiothreitol and sodium dithionite; Promega Corp., USA) was added and luminescence was measured after a minimum of 15 min. incubation at RT.

### Materials:

HeLa-R5-16 cells (cell line to express HIV gp160 upon doxycycline induction) are cultured in DMEM medium containing nutrients and 10 % (v/v) FCS with 400 µg/ml G418 and 200 µg/ml Hygromycin B.

CEM.NKR-CCR5-Luc (Catalog Number: 5198) is a T-cell line available from NIH AIDS Research & Reference Reagent Program McKesson BioServices Corporation Germantown, MD 20874, USA. Cell Type: CEM.NKR-CCR5 (Cat. #4376) was transfected (electroporation) to express the luciferase gene under the transcriptional control of the HIV-2 LTR and propagated in RPMI 1640 containing 10 % fetal bovine serum, 4 mM glutamine, penicillin/streptomycin and 0.8 mg/ml geneticin sulfate (G418). Growth Characteristics: Round lymphoid cells, morphology not very variable. Cells grow in suspension as single cells, which can form small clumps. Split 1:10 twice weekly. Special Characteristics: Express luciferase activity after transactivation of the HIV-2 LTR. Suitable for infection with primary HIV isolates, for neutralization and drug-sensitivity assays (Spenlehauer, C., et al., Virology 280 (2001) 292-300; Trkola, A., et al., J. Virol. 73 (1999) 8966-8974). The cell line was obtained through the NIH AIDS Research and Reference Reagent Program, NIAID, NIH from Drs. John Moore and Catherine Spenlehauer.

Bright-Glo™ Luciferase assay buffer (Promega Corp., USA, Part No E2264B) Bright-Glo™ Luciferase assay substrate (Promega Corp., USA, part No EE26B)

### Results:

The results are presented in Table 2. IC₅₀ values are between 46 and 399 ng/ml. The antibody's constant region is a mutated IgG1 (IgG1v2).

**Table 2:**

| heavy chain variable domain | light chain variable domain | IC₅₀ [ng/ml] |
|---|---|---|
| SEQ ID NO: 6 | SEQ ID NO: 9 | 108 |
| SEQ ID NO: 6 | SEQ ID NO: 10 | 399 |
| SEQ ID NO: 7 | SEQ ID NO: 9 | 46 |
| SEQ ID NO: 7 | SEQ ID NO: 10 | 152 |
| SEQ ID NO: 8 | SEQ ID NO: 9 | 132 |
| SEQ ID NO: 8 | SEQ ID NO: 10 | 76 |

### Example 4

### Antiviral assay with live virus

PBMCs were prepared from buffy coat isolated by density-gradient centrifugation using Lymphoprep™ (Nycomed Pharma AG, Oslo, Norway). Cells from four different donors were mixed, stimulated for 1 day with PHA and subsequently cultured in RPMI medium containing 1 % (w/v) penicillin/streptomycin, 1 % GlutaMAX™ (Invitrogen Corp., USA, Cat. No. 35050-038), 1 % sodium pyruvate, 1 % (w/v) non-essential amino acids and 10 % FBS, for two days in the presence of 5 U/ml IL-2 (interleukin-2).

100,000 PBMC (peripheral blood mononuclear cells) in 50 µl were added to 100 µl of an antibody solution (serial dilution ranged between 0.006-17.5 µg/ml, in supplemented RPMI medium and infected with 250 TCID50 (median tissue culture infective dose) of NLBal (NL4.3 strain (Adachi, A., et al., J. Virol. 59 (1986) 284-291) with the env of BaL (gp120)) or alternatively JRCSF (O'Brien, W.A., et al., Nature 348 (1990) 69-73) in a volume of 50 µl. The mixture was incubated for 6 days at 37 °C in a CO2 incubator. The supernatant was harvested and subsequently diluted 1:50 with 5 U/ml IL-2 supplemented RPMI medium.

Measurement of p24 was performed by a HIV-1 p24 ELISA (Perkin-Elmer, USA). The samples were then neutralized and transferred to microplate wells which were coated with a highly specific mouse monoclonal antibody to HIV-1 p24. The immobilized monoclonal antibody captures HIV-1 p24. Cell culture samples do not require disruption and were added directly to the monoclonal antibody-coated microplate wells. The captured antigen is complexed with biotinylated polyclonal antibody to HIV-1 p24, followed by a Streptavidin-HRP (horseradish peroxidase) conjugate. The resulting complex was detected by incubation with ortho-phenylenediamine-HCl (OPD) which produces a yellow color that is directly proportional to the amount of HIV-1 p24 captured. The absorbance of each microplate well was determined using a microplate reader and calibrated against the absorbance of an HIV-1 p24 antigen standard or standard curve.

### Results:

For the inhibition of HIV growth in human PBMC IC50 values in the range of from 2.27 ng/ml to 14.21 ng/ml for anti-CCR5 antibodies comprising the different combinations of heavy (SEQ ID NO:6, 7, 8) and light (SEQ ID NO:9, 10) chain variable domains and of an IgG1 isotype have been determined. For these combinations the IC90 values are in the range of from 9.77 ng/ml to 74.06 ng/ml.

For anti-CCR5 antibodies comprising a mutated IgG1 constant region (IgG1v2) the IC50 values of the different combinations of heavy and light chain variable domains have been determined to be in the range of from 8.22 ng/ml to 43.11 ng/ml whereas the IC90 values were determined to be in the range of from 51.95 ng/ml to 311.38 ng/ml.

### Example 5

### CCR5 Cell ELISA

20,000 CHO cells recombinantly expressing CCR5 were seeded per 96 well plate, and incubated overnight at 37 °C. Thereafter medium was aspirated and 40 µl fresh medium was added. 10 µl in medium diluted first antibody was added and incubated two hours at 4 °C. Medium was aspirated, 100 µl glutardialdehyde (c = 0.05 % in phosphate buffered saline (PBS)) was added and incubated 10 min. at room temperature. After washing three times with 200 µl PBS, 50 µl detection antibody (1:1,000 to 1:2,000 diluted in ELISA blocking buffer) was added and incubated two hours at room temperature. 50 µl 3,3',5,5'-tetramethylbenzidine (TMB) was added and the reaction is stopped after 7 min. Optical Density was measured at 450 nm (versus 620 nm).

First antibody: antibody to be examined
Second (detection) antibody: Sheep anti-human-IgG-gamma specific peroxidase-conjugated antibody (The Binding site Cat. # AP004) 1:2,000 (6 µl/12 ml) diluted in PBS 10 % blocking buffer
Medium: HAM's F-12 or GIBCO with GlutaMAX™, 10 % FCS, 200 µg/ml Hygromycin (Roche Diagnostics GmbH, Germany)
ELISA-Blocking: Roche Diagnostics GmbH, Germany, #1112589, 10 % (v/v) solution in water, 1:10 diluted in PBS
TMB: Roche Diagnostics GmbH, Germany, #1432559, solution for use

Results:
The results of the CCR5 cell ELISA shows that the binding to human CCR5 of anti-CCR5 antibodies comprising the different combinations of heavy (SEQ ID NO:6, 7, 8) and light (SEQ ID NO:9, 10) chain variable domains is in the range of from 2.71 to 3.13 (OD 450/620) at a concentration of 1000 ng/ml.

### Example 6

### Potential of CCR5 MAbs to bind to FcγRIIIa on NK cells

To determine the ability of the antibodies of the invention to bind to FcγRIIIa (CD16) on Natural Killer (NK) cells, Peripheral Blood Mononuclear Cells (PBMCs) are isolated and incubated with 20 µg/ml of antibody and control antibodies in the presence or absence of 20 µg/ml of a blocking mouse antibody to FcγRIIIa (anti-CD16, done 3G8, RDI, Flanders, NJ), to verify binding via FcγRIIIa. As negative controls, human IgG2 and IgG4 (The Binding Site), that do not bind FcγRIIIa, are used. Human IgG1 and IgG3 (The Binding Site) are included as positive controls for FcγRIIIa binding. Bound antibodies on NK cells are detected by FACS analysis using a PE-labeled mouse anti-human CD56 (NK-cell surface marker) antibody (BD Biosciences Pharmingen, San Diego, USA) in combination with a EITC-labeled goat F(ab)₂ anti-human IgG (Fc) antibody (Protos Immunoresearch, Burlingame, USA). Maximum binding (Bₘₐₓ) is determined at an antibody concentration of 20 µg/ml. Control antibody (human IgG4) shows up to 30 % Bₘₐₓ compared to 100 % Bₘₐₓ for human IgG1. Therefore "no FcγRIIIa binding or no ADCC" means at an antibody concentration of 20 µg/ml a Bₘₐₓ value of up to 30 % compared to human IgG1.

### Example 7

### CCR5 Chemotaxis Assay

L1.2hCCR5 cells were cultured in RPMI 1640 containing 10 % Fetal bovine serum, 1 x Penicillin/Streptomycin, 1 x glutamine, 1 x sodium pyruvate, 1 x β-mercaptoethanol, and 250 µg/ml G418 (all from Invitrogen Inc., USA). Just prior to the set up of the chemotaxis assay, the cells were spun down and resuspended in Chemotaxis Buffer (Hank's Balanced Salt Solution HBSS (Invitrogen) containing 0.1 % BSA and 10 mM HEPES). The cells were used in the chemotaxis assay at a final concentration of 5 x 10⁶ cells/ml. The CCR5 ligands human MIP1α, human MIP1β or human RANTES (R&D Systems, USA) were diluted in Chemotaxis Buffer and used at a final concentration of 20 nM. Test antibodies or the appropriate isotype control antibodies were diluted in HBSS. The chemotaxis assay was set up in the 0.5 µm pore 96-well ChemoTx^{®} system (Neuroprobe Inc., USA). Each antibody was mixed with one of the CCR5 ligands and 30 µl of this mixture was placed in the bottom well of the ChemoTx^{®} system. The filter screen was placed on top of the bottom wells. Each antibody was mixed with the L1.2hCCR5 cells and 20 µl of this mixture was placed on the filter. The plates were then placed in a humidified chamber and incubated at 37 °C and 5 % CO₂ for three hours. After incubation, the cells were scraped off the filter and the plates were spun in a table top centrifuge at 2,000 rpm for 10 min. The filter was then removed and the density of the cells that have migrated to the bottom wells was detected using CyQUANT^{®} Cell proliferation assay kit (Invitrogen) and the Spectra MAX GeminiXS plate reader (Molecular Devices, Wokingham, UK) according to the manufacturers' instructions. IC₅₀ values were calculated using Prism 4 (GraphPad Inc., USA).

The IC₅₀ values for human MIP-1α, human MIP-1β, and human RANTES for the different combinations of heavy chain variable domains and light chain variable domains with an IgG1 isotype constant region are in the range of from 0.80 nM to 0.91 nM, of from 0.72 nM to 1.08 nM, and of from 0.85 nM to 2.69 nM, respectively.

In case of a mutated IgG1 isotype (IgG1v2) are the IC₅₀ values for human MIP-1α, human MIP-1β, and human RANTES in the range of from 2.21 nM to 6.28 nM, of from 2.16 nM to 6.87 nM, and of from 3.59 nM to 5.03 nM, respectively.

## Claims

1. Antibody binding to CCR5 **characterized in that** the heavy chain variable domain comprises an amino acid sequence of SEQ ID NO: 1 wherein X01 is Gln, X02 is Glu, X03 is Lys, X04 is Leu or Pro, X05 is Lys, X06 is Ile or Thr, X07 is Thr, X08 is Thr, and
**characterized in that** the light chain variable domain comprises an amino acid sequence of SEQ ID NO: 2 wherein X10 is Ala, X11 is Tyr, X12 is Pro, X13 is Ala, X14 is Lys, X15 is Val or Ile, X16 is Gln, X17 is Ser, X18 is Leu, X19 is Lys, X20 is Asn, X21 is Ala, X22 is Gly, X23 is Asn, X24 is Val.

2. Antibody binding to CCR5 according to claim 1 or a CCR5-binding fragment thereof, **characterized in that** said antibody or CCR5-binding fragment thereof comprises a heavy chain variable domain selected from the heavy chain variable domains of SEQ ID NO: 6, 7, or 8.

3. Antibody binding to CCR5 according to any one of the preceding claims or a CCR5-binding fragment thereof, **characterized in that** said antibody or CCR5-binding fragment thereof comprises a light chain variable domain selected from the light chain variable domains of SEQ ID NO: 9, or 10.

4. Antibody binding to CCR5 according to any one of claims 2 or 3 or a CCR5-binding fragment thereof, **characterized in that** said antibody or CCR5-binding fragment thereof comprises a heavy chain variable domain selected from SEQ ID NO: 6, 7, or 8, or a CCR5-binding fragment thereof, and in comprising a light chain variable domain selected from SEQ ID NO: 9, or 10, wherein said heavy and light chain variable domains are selected independently of each other.

5. Antibody binding to CCR5 according to any one of the preceding claims, **characterized in that** said heavy chain constant region is of human IgG4 isotype or is of human IgG1 isotype modified in the hinge region at amino acid position 216-240 between C_{H}1 and C_{H}2, and/or in the second inter-domain region at amino acid position 327-331 between C_{H}2 and C_{H}3.

6. Antibody binding to CCR5 according to any one of the preceding claims, **characterized in that** said antibody comprises a heavy chain constant region of SEQ ID NO: 3 or 4, and comprises a light chain constant region of SEQ ID NO: 5.

7. Antibody binding to CCR5 according to any one of claims 5 to 6, **characterized in that** the antibody is of human IgG1 isotype and comprises the mutations L234A and L235A, or the antibody is of human IgG4 isotype comprising the mutation S228P.

8. Pharmaceutical composition comprising an antibody according to claim 1.

9. Method for the manufacture of a pharmaceutical composition comprising a pharmaceutically effective amount of an antibody according to claim 1.

10. Antibody according to claim 1 for use for the treatment of HIV infection.

11. Use of an antibody according to claim 1 for the manufacture of a medicament for the treatment of HIV infection.

12. Nucleic acid encoding an antibody binding to CCR5 according claim 1.

13. Method for the production of a recombinant human or humanized antibody according to claim 1, **characterized in that** a nucleic acid encoding an antibody binding to CCR5 according to claim 1 is expressed in a prokaryotic or eukaryotic host cell and said recombinant antibody is recovered from said cell or the cell culture supernatant.

14. Eukaryotic cell comprising a nucleic acid according to claim 12.

## Patentansprüche

1. Antikörper, der an CCR5 bindet, **dadurch gekennzeichnet, dass** die variable Domäne der schweren Kette eine Aminosäuresequenz der SEQ ID NO:1 umfasst, wobei X01 Gln ist, X02 Glu ist, X03 Lys ist, X04 Leu oder Pro ist, X05 Lys ist, X06 Ile oder Thr ist, X07 Thr ist, X08 Thr ist, und
**dadurch gekennzeichnet, dass** die variable Domäne der leichten Kette eine Aminosäuresequenz der SEQ ID NO:2 umfasst, wobei X10 Ala ist, X11 Tyr ist, X12 Pro ist, X13 Ala ist, X14 Lys ist, X15 Val oder Ile ist, X16 Gln ist, X17 Ser ist, X18 Leu ist, X19 Lys ist, X20 Asn ist, X21 Ala ist, X22 Gly ist, X23 Asn ist, X24 Val ist.

2. Antikörper, der an CCR5 bindet, gemäß Anspruch 1 oder ein CCR5 bindendes Fragment davon, **dadurch gekennzeichnet, dass** der Antikörper oder das CCR5 bindende Fragment davon eine variable Domäne der schweren Kette ausgewählt aus den variablen Domänen der schweren Kette der SEQ ID NO:6, 7 oder 8 umfasst.

3. Antikörper, der an CCR5 bindet, gemäß einem der vorherigen Ansprüche oder ein CCR5 bindendes Fragment davon, **dadurch gekennzeichnet, dass** der Antikörper oder das CCR5 bindende Fragment davon eine variable Domäne der leichten Kette ausgewählt aus den variablen Domänen der leichten Kette der SEQ ID NO:9 oder 10 umfasst.

4. Antikörper, der an CCR5 bindet, gemäß einem der Ansprüche 2 oder 3 oder ein CCR5 bindendes Fragment davon, **dadurch gekennzeichnet, dass** der Antikörper oder das CCR5 bindende Fragment davon eine variable Domäne der schweren Kette ausgewählt aus der SEQ ID NO:6, 7 oder 8 oder ein CCR5 bindendes Fragment davon und eine variable Domäne der leichten Kette ausgewählt aus der SEQ ID NO:9 oder 10 umfasst, wobei die variablen Domänen der schweren und leichten Kette unabhängig von einander ausgewählt sind.

5. Antikörper, der an CCR5 bindet, gemäß einem der vorherigen Ansprüche, **dadurch gekennzeichnet, dass** die konstante Region der schweren Kette von dem menschlichen IgG4-Isotyp oder von dem menschlichen IgG1-Isotyp ist, welcher in der Gelenkregion an den Aminosäurepositionen 216-240 zwischen C_{H}1 und C_{H}2 und/oder in der zweiten Interdomäneregion an den Aminosäurepositionen 327-331 zwischen C_{H}2 und C_{H}3 modifiziert ist.

6. Antikörper, der an CCR5 bindet, gemäß einem der vorherigen Ansprüche, **dadurch gekennzeichnet, dass** der Antiköper eine konstante Region der schweren Kette der SEQ ID NO:3 oder 4 und eine konstante Region der leichten Kette der SEQ ID NO:5 umfasst.

7. Antikörper, der an CCR5 bindet, gemäß einem der Ansprüche 5 bis 6, **dadurch gekennzeichnet, dass** der Antikörper vom menschlichen IgG1-Isotyp ist und die Mutationen L234A und L235A umfasst, oder der Antikörper vom menschlichen IgG4-Isotyp, umfassend die Mutation S228P, ist.

8. Arzneimittel, umfassend einen Antikörper gemäß Anspruch 1.

9. Verfahren zur Herstellung eines Arzneimittels, umfassend eine pharmazeutisch wirksame Menge eines Antikörpers gemäß Anspruch 1.

10. Antikörper gemäß Anspruch 1 zur Verwendung in der Behandlung einer HIV-Infektion.

11. Verwendung eines Antikörpers gemäß Anspruch 1 für die Herstellung eines Medikaments zur Behandlung einer HIV-Infektion.

12. Nucleinsäure, welche einen Antikörper gemäß Anspruch 1, der an CCR5 bindet, codiert.

13. Verfahren zur Herstellung eines rekombinanten menschlichen oder humanisierten Antikörpers gemäß Anspruch 1, **dadurch gekennzeichnet, dass** eine Nucleinsäure, welche einen Antikörper codiert, der an CCR5 bindet, gemäß Anspruch 1 in einer prokaryotischen oder eukaryotischen Wirtselle exprimiert wird und der rekombinante Antikörper von der Zelle oder dem Zellkulturüberstand gewonnen wird.

14. Eukaryotische Zelle, umfassend eine Nucleinsäure gemäß Anspruch 12.

## Revendications

1. Anticorps se liant au CCR5, **caractérisé en ce que** le domaine variable de la chaîne lourde comprend une séquence d'aminoacides de SEQ ID NO: 1: où X01 est Gln, X02 est Glu, X03 est Lys, X04 est Leu ou Pro, X05 est Lys, X06 est Ile ou Thr, X07 est Thr, X08 est Thr et
**caractérisé en ce que** le domaine variable de la chaîne légère comprend une séquence d'aminoacides de SEQ ID NO: 2: où X10 est Ala, X11 est Tyr, X12 est Pro, X13 est Ala, X14 est Lys, X15 est Val ou Ile, X16 est Gln, X17 est Ser, X18 est Leu, X19 est Lys, X20 est Asn, X21 est Ala, X22 est Gly, X23 est Asn, X24 est Val.

2. Anticorps se liant au CCR5 selon la revendication 1 ou un de ses fragments se liant au CCR5, **caractérisé en ce que** ledit anticorps ou son fragment se liant au CCR5 comprend un domaine variable de chaîne lourde choisi parmi les domaines variables de chaîne lourde de SEQ ID NO: 6, 7 ou 8.

3. Anticorps se liant au CCR5 selon l'une quelconque des revendications précédentes ou un de ses fragments se liant au CCR5, **caractérisé en ce que** ledit anticorps ou son fragment se liant au CCR5 comprend un domaine variable de chaîne légère choisi parmi les domaines variables de chaîne légère de SEQ ID NO: 9 ou 10.

4. Anticorps se liant au CCR5 selon l'une quelconque des revendications 2 ou 3 ou un de ses fragments se liant au CCR5, **caractérisé en ce que** ledit anticorps ou son fragment se liant au CCR5 comprend un domaine variable de chaîne lourde choisi parmi SEQ ID NO: 6, 7 ou 8, ou l'un de ses fragments se liant au CCR5 et **en ce qu'**il comprend un domaine variable de chaîne légère choisi parmi SEQ ID NO: 9 ou 10, lesdits domaines variables de chaîne lourde et légère étant choisis indépendamment l'un de l'autre.

5. Anticorps se liant au CCR5 selon l'une quelconque des revendications précédentes, **caractérisé en ce que** ladite région constante de chaîne lourde est de l'isotype IgG4 humain ou est de l'isotype IgG1 humain modifiés dans la région charnière au niveau des positions d'aminoacides 216-240 entre C_{H}1 et C_{H}2, et/ou dans la deuxième région interdomaniale au niveau des positions d'aminoacides 327-331 entre C_{H}2 et C_{H}3.

6. Anticorps se liant au CCR5 selon l'une quelconque des revendications précédentes, **caractérisé en ce que** ledit anticorps comprend une région constante de chaîne lourde de SEQ ID NO: 3 ou 4, et comprend une région constante de chaîne légère de SEQ ID NO: 5.

7. Anticorps se liant au CCR5 selon l'une quelconque des revendications 5 ou 6, **caractérisé en ce que** l'anticorps est de l'isotype IgG1 humain et comprend les mutations L234A et L235A, ou l'anticorps est de l'isotype IgG4 humain comprenant la mutation S228P.

8. Composition pharmaceutique comprenant un anticorps selon la revendication 1.

9. Procédé de préparation d'un composition pharmaceutique comprenant une quantité pharmaceutiquement efficace d'un anticorps selon la revendication 1.

10. Anticorps selon la revendication 1, à utiliser pour le traitement d'une infection par le VIH.

11. Utilisation d'un anticorps selon la revendication 1 pour la fabrication d'un médicament destiné au traitement d'une infection par le VIH.

12. Acide nucléique codant pour un anticorps se liant au CCR5 selon la revendication 1.

13. Procédé de production d'un anticorps humain ou humanisé recombinant selon la revendication 1, **caractérisé en ce qu'**un acide nucléique codant pour un anticorps se liant au CCR5 selon la revendication 1 est exprimé dans une cellule hôte procaryote ou eucaryote et on récupère ledit anticorps recombinant à partir de ladite cellule ou du surnageant de culture de la cellule.

14. Cellule eucaryote comprenant un acide nucléique selon la revendication 12.


### SEQUENCE LISTING

<110> F. Hoffmann-La Roche AG
<120> Antibodies against CCR5 and uses thereof
<130> 23961 WO
<150> EP 06020646.3
   <151> 2006-09-29
<160> 10
<170> PatentIn version 3.2
<210> 1
   <211> 117
   <212> PRT
   <213> mouse
<220>
   <221> misc_feature
   <222> (5) .. (5)
   <223> Xaa is X01 is Lys or Gln
<220>
   <221> misc_feature
   <222> (6) .. (6)
   <223> Xaa is X02 is Gln or Glu
<220>
   <221> misc_feature
   <222> (13) .. (13)
   <223> Xaa is X03 is Arg or Lys
<220>
   <221> misc_feature
   <222> (45) .. (45)
   <223> Xaa is X04 is Leu or Pro
<220>
   <221> misc_feature
   <222> (64) .. (64)
   <223> Xaa is X05 is Met or Lys
<220>
   <221> misc_feature
   <222> (92) .. (92)
   <223> Xaa is X06 is Ile or Thr
<220>
   <221> misc_feature
   <222> (112)..(112)
   <223> Xaa is X07 is Ser or Thr
<220>
   <221> misc_feature
   <222> (114) .. (114)
   <223> Xaa is X08 is Ile or Thr
<400> 1
   Gln Val Gln Leu Xaa Xaa Ser Gly Pro Gly Leu Val Xaa Pro Ser Gln
   1               5                   10                  15
   Ser Leu Ser Ile Thr Cys Thr Val Ser Gly Phe Pro Leu Gly Ala Phe
               20                  25                  30
   Gly Val His Trp Val Arg Gln Ser Pro Gly Lys Gly Xaa Glu Trp Leu
           35                  40                  45
   Gly Val Ile Trp Lys Gly Gly Asn Thr Asp Tyr Asn Ala Ala Phe Xaa
       50                  55                  60
   Ser Arg Leu Arg Ile Thr Lys Asp Asn Ser Lys Ser Gln Val Phe Phe
   65                  70                  75                  80
   Arg Met Asn Ser Leu Gln Thr Asp Asp Thr Ala Xaa Tyr Tyr Cys Ala
                   85                  90                  95
   Lys Val Asn Leu Ala Asp Ala Met Asp Tyr Trp Gly Gln Gly Thr Xaa
               100                 105                 110
   Val Xaa Val Ser Ser
           115
<210> 2
   <211> 107
   <212> PRT
   <213> mouse
<220>
   <221> misc_feature
   <222> (29) .. (29)
   <223> Xaa is X10 is Ile or Ala
<220>
   <221> misc_feature
   <222> (36) .. (36)
   <223> Xaa is X11 is Phe or Tyr
<220>
   <221> misc_feature
   <222> (40) .. (40)
   <223> Xaa is X12 is Gln or Pro
<220>
   <221> misc_feature
   <222> (43) .. (43)
   <223> Xaa is X13 is Ser or Ala
<220>
   <221> misc_feature
   <222> (45) .. (45)
   <223> Xaa is X14 is Gln or Lys
<220>
   <221> misc_feature
   <222> (48) .. (48)
   <223> Xaa is X15 is Val or Ile
<220>
   <221> misc_feature
   <222> (70) .. (70)
   <223> Xaa is X16 is Gln or Asp
<220>
   <221> misc_feature
   <222> (72) .. (72)
   <223> Xaa is X17 is Ser ot Thr
<220>
   <221> misc_feature
   <222> (73) .. (73)
   <223> Xaa is X18 is Leu or Ala
<220>
   <221> misc_feature
   <222> (74) .. (74)
   <223> Xaa is X19 is Lys or Thr
<220>
   <221> misc_feature
   <222> (76) .. (76)
   <223> Xaa is X20 is Asn or Ser
<220>
   <221> misc_feature
   <222> (78) .. (78)
   <223> Xaa is X21 is Leu or Ala
<220>
   <221> misc_feature
   <222> (84) .. (84)
   <223> Xaa is X22 is Gly or Ala
<220>
   <221> misc_feature
   <222> (85)..(85)
   <223> Xaa is X23 is Asn or Thr
<220>
   <221> misc_feature
   <222> (104)..(104)
   <223> Xaa is X24 is Leu or Val
<400> 2
   Asp Ile Gln Met Thr Gln Ser Pro Ala Ser Leu Ser Ala Ser Val Gly
   1               5                   10                  15
   Glu Thr Val Thr Ile Thr Cys Arg Ala Ser Gly Asn Xaa His Gly Tyr
               20                  25                  30
   Leu Ala Trp Xaa Gln Gln Lys Xaa Gly Lys Xaa Pro Xaa Leu Leu Xaa
           35                  40                  45
   Tyr Asn Thr Lys Thr Leu Ala Glu Gly Val Pro Ser Arg Phe Ser Gly
       50                  55                  60
   Ser Gly Ser Gly Thr Xaa Phe Xaa Xaa Xaa Ile Xaa Ser Xaa Gln Pro
   65                  70                  75                  80
   Glu Asp Phe Xaa Xaa Tyr Tyr Cys Gln His His Tyr Asp Leu Pro Arg
                   85                  90                  95
   Thr Phe Gly Gly Gly Thr Lys Xaa Glu Ile Lys
               100                 105
<210> 3
   <211> 330
   <212> PRT
   <213> Homo sapiens
<400> 3
<210> 4
   <211> 327
   <212> PRT
   <213> Homo sapiens
<400> 4
   Ala Ser Thr Lys Gly Pro Ser Val Phe Pro Leu Ala Pro Cys Ser Arg
   1 5 10 15
   Ser Thr Ser Glu Ser Thr Ala Ala Leu Gly Cys Leu Val Lys Asp Tyr
   20 25 30
   Phe Pro Glu Pro Val Thr Val Ser Trp Asn Ser Gly Ala Leu Thr Ser
   35 40 45
   Gly Val His Thr Phe Pro Ala Val Leu Gln Ser Ser Gly Leu Tyr Ser
   50 55 60
   Leu Ser Ser Val Val Thr Val Pro Ser Ser Ser Leu Gly Thr Lys Thr
   65 70 75 80
   Tyr Thr Cys Asn Val Asp His Lys Pro Ser Asn Thr Lys Val Asp Lys
   85 90 95
   Arg Val Glu Ser Lys Tyr Gly Pro Pro Cys Pro Ser Cys Pro Ala Pro
   100 105 110
   Glu Phe Leu Gly Gly Pro Ser Val Phe Leu Phe Pro Pro Lys Pro Lys
   115 120 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Arg Leu Thr Val Asp Lys Ser Arg Trp Gln Glu Gly Asn Val Phe Ser
       290                 295                 300
   Cys Ser Val Met His Glu Ala Leu His Asn His Tyr Thr Gln Lys Ser
   305                 310                 315                 320
   Leu Ser Leu Ser Leu Gly Lys
                   325
<210> 5
   <211> 107
   <212> PRT
   <213> Homo sapiens
<400> 5
<210> 6
   <211> 117
   <212> PRT
   <213> Artificial
<220>
   <223> heavy chain variable domain
<400> 6
   Gln Val Gln Leu Gln Glu Ser Gly Pro Gly Leu Val Lys Pro Ser Gln
   1               5                   10                  15
   Ser Leu Ser Ile Thr Cys Thr Val Ser Gly Phe Pro Leu Gly Ala Phe
               20                  25                  30
   Gly Val His Trp Val Arg Gln Ser Pro Gly Lys Gly Pro Glu Trp Leu
           35                  40                  45
   Gly Val Ile Trp Lys Gly Gly Asn Thr Asp Tyr Asn Ala Ala Phe Lys
       50                  55                  60
   Ser Arg Leu Arg Ile Thr Lys Asp Asn Ser Lys Ser Gln Val Phe Phe
   65                  70                  75                  80
   Arg Met Asn Ser Leu Gln Thr Asp Asp Thr Ala Thr Tyr Tyr Cys Ala
                   85                  90                  95
   Lys Val Asn Leu Ala Asp Ala Met Asp Tyr Trp Gly Gln Gly Thr Thr
               100                 105                 110
   Val Thr Val Ser Ser
           115
<210> 7
   <211> 117
   <212> PRT
   <213> Artificial
<220>
   <223> heavy chain variable domain
<400> 7
   Gln Val Gln Leu Gln Glu Ser Gly Pro Gly Leu Val Lys Pro Ser Gln
   1               5                   10                  15
   Ser Leu Ser Ile Thr Cys Thr Val Ser Gly Phe Pro Leu Gly Ala Phe
               20                  25                  30
   Gly Val His Trp Val Arg Gln Ser Pro Gly Lys Gly Leu Glu Trp Leu
           35                  40                  45
   Gly Val Ile Trp Lys Gly Gly Asn Thr Asp Tyr Asn Ala Ala Phe Lys
       50                  55                  60
   Ser Arg Leu Arg Ile Thr Lys Asp Asn Ser Lys Ser Gln Val Phe Phe
   65                  70                  75                  80
   Arg Met Asn Ser Leu Gln Thr Asp Asp Thr Ala Thr Tyr Tyr Cys Ala
                   85                  90                  95
   Lys Val Asn Leu Ala Asp Ala Met Asp Tyr Trp Gly Gln Gly Thr Thr
               100                 105                 110
   Val Thr Val Ser Ser
           115
<210> 8
   <211> 117
   <212> PRT
   <213> Artificial
<220>
   <223> heavy chain variable domain
<400> 8
   Gln Val Gln Leu Gln Glu Ser Gly Pro Gly Leu Val Lys Pro Ser Gln
   1               5                   10                  15
   Ser Leu Ser Ile Thr Cys Thr Val Ser Gly Phe Pro Leu Gly Ala Phe
               20                  25                  30
   Gly Val His Trp Val Arg Gln Ser Pro Gly Lys Gly Leu Glu Trp Leu
           35                  40                  45
   Gly Val Ile Trp Lys Gly Gly Asn Thr Asp Tyr Asn Ala Ala Phe Lys
       50                  55                  60
   Ser Arg Leu Arg Ile Thr Lys Asp Asn Ser Lys Ser Gln Val Phe Phe
   65                  70                  75                  80
   Arg Met Asn Ser Leu Gln Thr Asp Asp Thr Ala Ile Tyr Tyr Cys Ala
                   85                  90                  95
   Lys Val Asn Leu Ala Asp Ala Met Asp Tyr Trp Gly Gln Gly Thr Thr
               100                 105                 110
   Val Thr Val Ser Ser
           115
<210> 9
   <211> 107
   <212> PRT
   <213> Artificial
<220>
   <223> light chain variable domain
<400> 9
   Asp Ile Gln Met Thr Gln Ser Pro Ala Ser Leu Ser Ala Ser Val Gly
   1               5                   10                  15
   Glu Thr Val Thr Ile Thr Cys Arg Ala Ser Gly Asn Ala His Gly Tyr
               20                  25                  30
   Leu Ala Trp Tyr Gln Gln Lys Pro Gly Lys Ala Pro Lys Leu Leu Ile
           35                  40                  45
   Tyr Asn Thr Lys Thr Leu Ala Glu Gly Val Pro Ser Arg Phe Ser Gly
       50                  55                  60
   Ser Gly Ser Gly Thr Gln Phe Ser Leu Lys Ile Asn Ser Ala Gln Pro
   65                  70                  75                  80
   Glu Asp Phe Gly Asn Tyr Tyr Cys Gln His His Tyr Asp Leu Pro Arg
                   85                  90                  95
   Thr Phe Gly Gly Gly Thr Lys Val Glu Ile Lys
               100                 105
<210> 10
   <211> 107
   <212> PRT
   <213> Artificial
<220>
   <223> light chain variable domain
<400> 10
   Asp Ile Gln Met Thr Gln Ser Pro Ala Ser Leu Ser Ala Ser Val Gly
   1               5                   10                  15
   Glu Thr Val Thr Ile Thr Cys Arg Ala Ser Gly Asn Ala His Gly Tyr
               20                  25                  30
   Leu Ala Trp Tyr Gln Gln Lys Pro Gly Lys Ala Pro Lys Leu Leu Val
           35                  40                  45
   Tyr Asn Thr Lys Thr Leu Ala Glu Gly Val Pro Ser Arg Phe Ser Gly
       50                  55                  60
   Ser Gly Ser Gly Thr Gln Phe Ser Leu Lys Ile Asn Ser Ala Gln Pro
   65                  70                  75                  80
   Glu Asp Phe Gly Asn Tyr Tyr Cys Gln His His Tyr Asp Leu Pro Arg
                   85                  90                  95
   Thr Phe Gly Gly Gly Thr Lys Val Glu Ile Lys
               100                 105